# EUROPÄISCHE PATENTSCHRIFT

(11) **EP 1 260 234 B1**
(45) Veröffentlichungstag und Bekanntmachung des Hinweises auf die Patenterteilung: **13.04.2005**
(21) Anmeldenummer: 02009079.1
(22) Anmeldetag: 24.04.2002
(51) Int. Cl.: A61L 2/10, A61L 2/18, A61L 2/24, A61B 19/00

(54) **Verfahren zur Entkeimung von als Spülflüssigkeit für einen programmgesteuerten Spülautomaten benutztes Frisch- oder Brauchwasser**
Process for sterilizing fresh or industrial water used as rinsing fluid for an automated washing machine
Procédé pour stériliser l'eau potable ou industrielle utilisée comme liquide de rinçage dans une machine à laver automatique

(30) Priorität: 21.05.2001 DE 10124816
(43) Veröffentlichungstag der Anmeldung: 27.11.2002
(73) Patentinhaber: Miele & Cie. KG, 33332 Gütersloh (DE)
(72) Erfinder: Leifeld, Ludger, 59227 Ahlen (DE); Michels, Winfried, Dr., 34414 Warburg (DE)

(56) Entgegenhaltungen:
- DE-A- 2 220 189
- DE-A- 3 327 466
- DE-A- 4 110 687
- DE-A- 4 342 573
- US-A- 4 289 728

## Beschreibung

Verfahren zur Entkeimung von als Spülflüssigkeit für einen programmgesteuerten Spülautomaten benutztes Frisch- oder Brauchwasser Die Erfindung betrifft ein Verfahren zur Entkeimung von Frisch- oder Brauchwasser, welches als Spülflüssigkeit in einem programmgesteuerten Spülautomaten zur chemothermischen Desinfektion von medizinischen Instrumenten, wie Endoskope oder dergl. verwendet wird, wobei die Spülflüssigkeit jeweils in den wasserführenden Programmabschnitten Vorspülen, Reinigen, Zwischenspülen und Instrumentendesinfektion oder Nachspülen dem Spülbehälter des Automaten ggf. über einen Enthärter zugeführt und zur Vermeidung einer Rekontamination beim Nachspülen mit UV-Licht bestrahlt wird.

Die maschinelle Dekontamination chirurgischer Instrumente erfordert eine optimale Reinigung und Desinfektion. Die im Labor und Krankenhaus eingesetzten Spülautomaten sind hierfür mit Spezialprogrammen für die Behandlung des unterschiedlichen medizinischen Instrumentariums ausgestattet. Aus der DE 33 27 466 A1 ist ein Verfahren zum Reinigen, Spülen und Desinfizieren von Labor- und Operationsgegenständen bekannt, bei dem alle Verfahrensschritte unter bedarfsweiser Beigabe von Reinigungs- und Desinfektionsmittel zur Schonung des Reinigungsgutes in einem Temperaturbereich von 60° C bis 70° C durchgeführt werden. Von der Anmelderin sind ebenfalls Spülautomaten bekannt (z. B. Miele G7735/7736), die auf dem Gebiet der thermischen und/oder chemischen Desinfektion und Reinigung zur maschinellen Aufbereitung von medizinischem und chirurgischem Instrumentarium eingesetzt werden. Zu diesem Instrumentarium gehören beispielsweise in der Minimal-Invasiven Chirurgie (MIC) benutzte Instrumente, z. B. starre Endoskope und dergl. Für die Aufbereitung solcher Instrumente ist aus der DE-A 43 42 573 ein Dekontaminations-Spülverfahren für Endoskope bekannt, welches hinsichtlich seiner Modifikation bezüglich der Kompatibilität gegenüber Optiken und auch für die materialkompatible Aufbereitung von Patientensystemen eine sehr gute Reinigungsleistung besitzt. Ferner ist der DE 38 16 734 A1 ein Reinigungs- und Desinfektionsverfahren für hitzeund korrosionsempfindliche medizinische Geräte zu entnehmen, bei dem zur Schonung des Reinigungsgutes in einem Temperaturbereich von 55° bis 65°C und unter Beigabe von Reinigungs- und Desinfektionsmittel mit weichem Wasser gereinigt, desinfiziert und gespült wird.

DE-A-22 20 189 betrifft eine Geschirrspülmaschine mit einem Enthärter, bei welcher das Geschirr auch nach längeren Benutzungspausen mit keimfreiem Wasser gespült wird. Zu diesem Zweck wird vorgeschlagen, dass in der Geschirrspülmaschine in der Wasserzuleitung ein UV - Strahler angeordnet ist.

Bei einem von der Anmelderin benutzten chemothermischen Verfahren zur Aufbereitung flexibler Endoskope wird in einem Spülprogrammabschnitt Desinfektion die mit einem Desinfektionsmittel (z. B. Glutardialdehyd) versetzte Spülflüssigkeit auf 60 °C erwärmt und diese Temperatur ca. 5 min. wirksam eingehalten. Anschließend wird zur Beseitigung der Desinfektionswirkstoffe in mehreren Schritten nachgespült und im letzten Schritt die Nachspülflüssigkeit erwärmt. Durch die Erwärmung der Spülflüssigkeit im letzten Programmschritt auf 60° C zuzüglich einer vorgegebenen Einwirkzeit wird ein Großteil aller Wasserbakterien, wie Pseudomonas aeruginosa abgetötet. Die Desinfektionssicherheit ist dabei jedoch sehr begrenzt und stark abhängig vom Verkeimungsgrad der zugeführten Spülflüssigkeit. Der Verkeimungsgrad hängt wiederum mit der Stillstandzeit des Spülautomaten und der Verweilzeit des Wassers im geräteeigenen Enthärter ab, in welchem - wie allgemein bekannt - ein besonders massives Wachstum der Bakterien stattfindet. Unabhängig davon ist eine Bakterienbelastung auch von der Hygiene des Hauswassernetzes abhängig. Über den zuvor genannten Keim hinaus, der thermisch leicht zu vernichten ist, sind in aller Regel jedoch auch Bakterien vorhanden, die resistenter gegenüber der feuchten Hitze sind. Hier sind insbesondere atypische Mykobakterien oder aber auch Legionellen, die bei 60 °C kaum erfasst werden, zu nennen. Um hier mehr Desinfektionssicherheit zu erzeugen und die flexiblen Endoskope im Spülverlauf nicht wieder-zu rekontaminieren, ist bei der Anmelderin auch schon versucht worden, im Wasserkreislauf der Spülflüssigkeit vor dem Einlauf der Spülflüssigkeit in den Spülbehälter einen UV-Strahler als Desinfektionseinheit anzuordnen, der bevorzugt bei einer Wellenlänge von 254 nm arbeitet. Das vom Strahler ausgesandte UV-Licht wirkt zerstörend auf die DNA der Bakterienzellen. Auch wurde bei der Verwendung von UV-behandelter Spülflüssigkeit versucht, auf das Aufheizen der Flüssigkeit im letzten Spülschritt zu verzichten. Bei einer Überprüfung des Nachspülwassers stellte sich jedoch heraus, dass trotz der Wasserbehandlung dennoch Keime festzustellen waren und die UV-Lampe allein nicht ausreicht. Dies kann offenbar darauf zurückzuführen sein, dass insbesondere bei etwas trüberem Wasser die Durchdringungstiefe des UV-Lichtes nicht gegeben ist, so dass Bakterienverbände und Konglomerate nur teilweise abgetötet werden. Die Kombination der UV-Lampe und das Aufheizen in der letzten Spülphase wurde somit mit dem vorbeschriebenen Nachteil der Verlängerung der Chargenzeit problemlösend wieder eingeführt.

Ausgehend von dem eingangs beschriebenen Verfahren zur Entkeimung der bei der Instrumentenaufbereitung im Spülautomaten eingesetzten Spülflüssigkeit liegt der Erfindung die Aufgabe zugrunde, die Desinfektionssicherheit des Spülautomaten zu erhöhen.

Erfindungsgemäß wird diese Aufgabe mit den Merkmalen des Patentanspruchs 1 gelöst. Vorteilhafte Ausgestaltungen und Weiterbildungen der Erfindung ergeben sich aus den nachfolgenden Unteransprüchen.

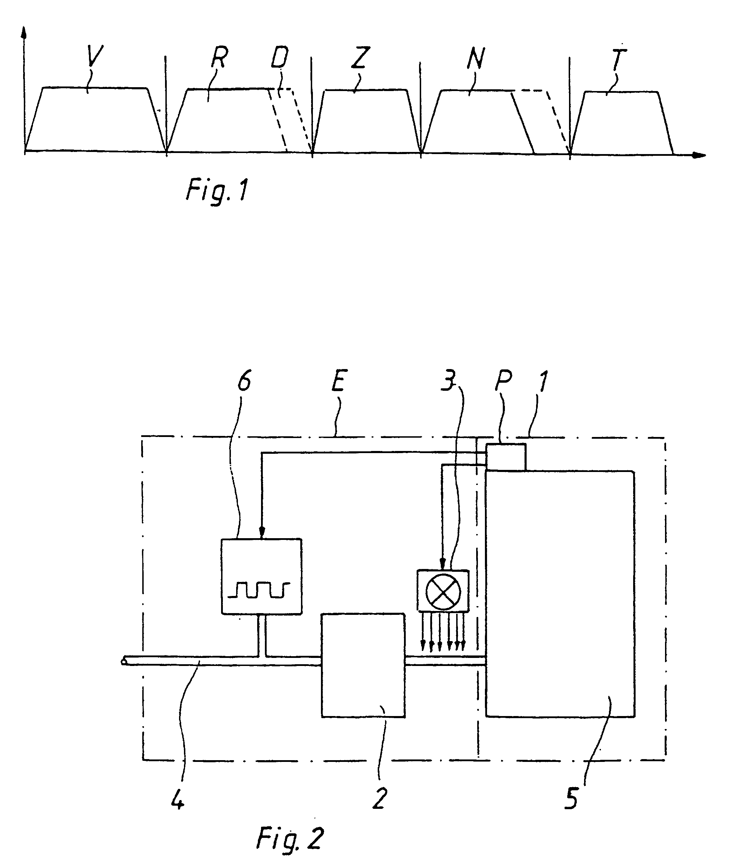

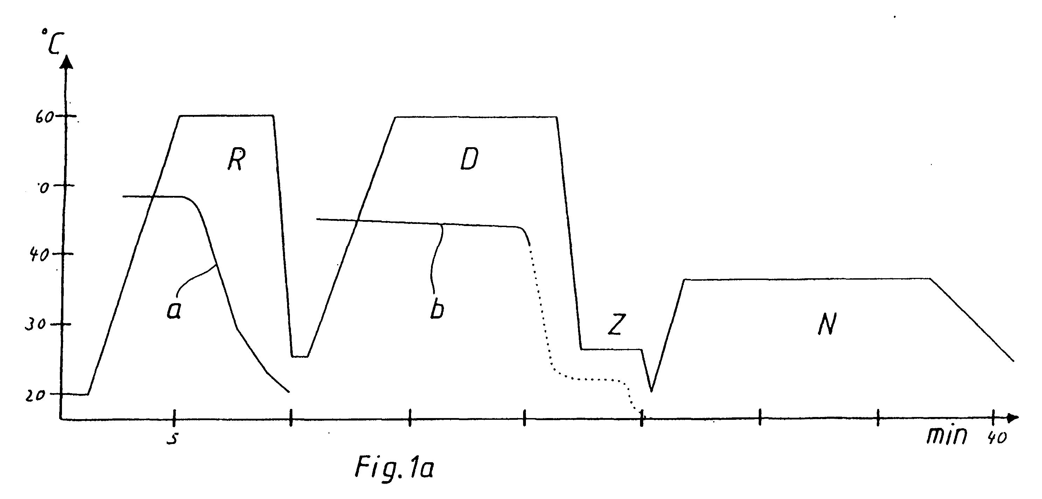

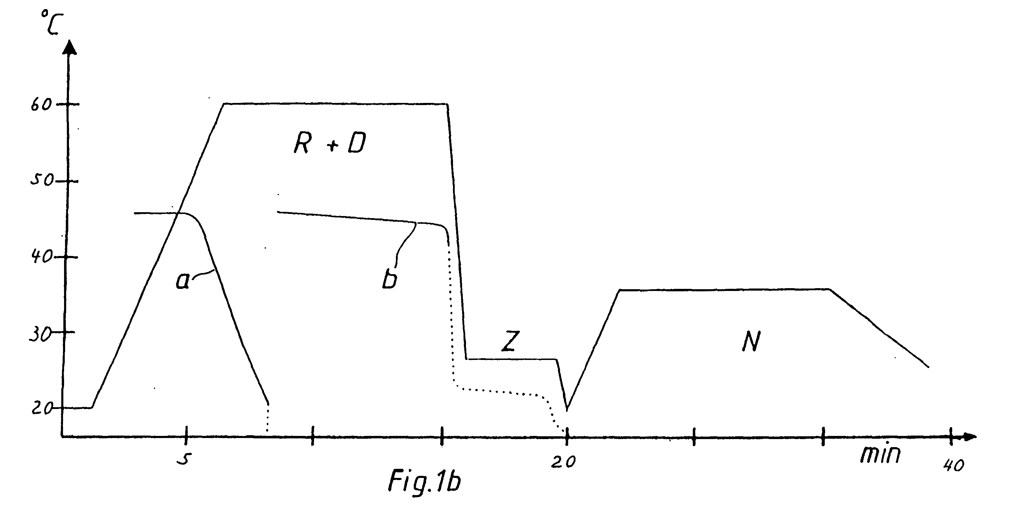

Ein Ausführungsbeispiel der Erfindung ist in den Zeichnungen rein schematisch dargestellt und wird nachfolgend näher beschrieben. Es zeigt
- Figur 1: den Programmablauf eines Spülautomaten für eine chemothermische Desinfektion von medizinischen Instrumenten,
- Figur 1a: ein für die Endoskopaufbereitung zugeschnittenes Desinfektionsprogramm,
- Figur 1 b: ein weiteres Programm für eine Endoskopaufbereitung,
- Figur 2: die Spülwasserzuführung zum Spülautomaten mit einer Desinfektionseinrichtung in vereinfachter Darstellung.

Für das erfindungsgemäße Verfahren wird ein Spülautomat (z. B. Miele G7735) verwendet, welcher auswählbare Programme zum Reinigen, Desinfizieren und ggf. Trocknen von im Labor und Krankenhaus benutzten medizinischen und/oder chirurgischen und endoskopischen Instrumenten, wie Operationsbestecken, Katheter, Atemschläuche, Endoskope und dergl. besitzt. Die zu behandelnden Instrumente werden dabei in an sich bekannter Weise in einem separaten Einsatz oder Spülgutträger des Spülautomaten abgelegt, wobei Reinigungsflüssigkeit den Einsatzwagen samt Spülgut in den wasserführenden Programmabschnitten durchströmt. Das Verfahren kann aber auch bei Gerätschaften zum Einsatz kommen, die in ihrem Aufbau und Gerätezubehör speziell auf die Reinigung und Desinfektion ganz bestimmter Instrumente, beispielsweise flexible Endoskope, abgestimmt sind. In aller Regel arbeiten verfahrensgemäß auch solche Spezial-Spülautomaten mit wasserführenden Spülprogrammabschnitten, wie Vorspülen (V), Reinigen (R), Desinfizieren (D), Zwischenspülen (Z) und Nachspülen (N), sh. Fig. 1, wobei ggf. über einen Enthärter zugeführtes Frisch- oder Brauchwasser aus dem örtlichen Leitungs- oder Brauchwassernetz, wie noch in Fig.2 näher beschrieben, als Spülflüssigkeit eingesetzt wird. Dem Programmabschnitt Nachspülen oder Schlussspülen (N) kann sich ggf. noch ein Programmabschnitt Trocknen (T) anfügen, in welchem das gereinigte Spülgut getrocknet wird.

Bis auf das Trocknen beinhalten die vorerwähnten Programmabschnitte Kalt- und Warmwasserspül- bzw. Reinigungsvorgänge, wobei die Spülflüssigkeitserwärmung über die nicht dargestellte geräteeigene Heizung des Spülautomaten erfolgt. Während im Vorspülgang (V) mit ka tem Frischwasser als Spülflüssigkeit gearbeitet wird, beinhaltet der Reinigungsgang (R) einen Warmspülgang, bei dem die Flüssigkeit auf 40°C bis 60°C erwärmt wird, wobei diese Temperatur über eine vorgegebene Zeit (Haltezeit) konstant gehalten wird. Nach der Instrumentendesinfektion laufen ggf. ein oder mehrere Zwischenspülgänge (Z) ab, die jeweils mit kalter Spülflüssigkeit durchgeführt werden. Den Abschluss der chemothermischen Instrumentenaufbereitung bildet das Nachspülen (N) oder Schlussspülen bei niedrig erwärmtem Spülwasser von vorzugsweise ca. 35° C. In den Reinigungs- und Desinfektionsspülgängen wird der verwendeten Spülflüssigkeit jeweits ein Reinigungs- bzw. Desinfektionsmittel beigegeben.

Die Fig. 1a zeigt beispielsweise ein Aufbereitungsprogramm (ohne Vorspülen V und Trocknen T) für Endoskope, wobei das Desinfizieren der Instrumente im Anschluss an das Reinigen (R) erfolgt. Bei Fig. 1 b hingegen ist die Desinfektionsphase (D) mit in den Programmabschnitt Re i-nigen verlegt, und zwar an das Ende dieses Abschnitts. Danach erfolgt das Zwischenspülen (Z) mit dem sich anschließenden Schlussspülen, welches bei Anwendung der Erfindung bei niedrig erwärmtem Nachspülwasser (ca. 35 C°) erfolgen kann. Mit (a) und (b) sind in den Fig. 1a, 1b jeweils die Spülprogrammphasen Reinigen und Desinfizieren bezeichnet, in denen programmgesteuert das Reinigungsmittel (z. B. ein enzymatischer Reiniger) bzw. das Mittel zur Instrumentendesinfektion (z. B. Glutardialdehyd) dem enthärteten und gemäß der Erfindung entkeimten Spülwasser im Spülbehälter zudosiert wird. Die erfindungsgemäße Spülwasserentkeimung ist nachstehend beschrieben.

Gemäß Fig. 2 ist vorgesehen, alles dem Spülautomaten (1) zugeführte Frischwasser bzw. sämtliche Spülflüssigkeit in den einzelnen Spülgängen zur Verringerung einer Keimbelastung nach dem Durchlauf durch einen Enthärter (2) mit UV-Licht zu bestrahlen. Das UV-Licht der vorgesehenen UV-Lampe (3) kann zur Abtötung wassergängiger Mikroorganismen im Brauchwasser, wie die fakultativ pathogenen Keime, Pseudomonas, Legionella und Mykobakterien eingesetzt werden. Die ultraviolette Strahlung wird von Quecksilber-Niederdruckstrahlern erzeugt, die hauptsächlich Licht einer bestimmten Wellenlänge (A=254nm) aussenden. Dieses Licht bewirkt photochemische Reaktionen an Zellbestandteilen (DNS) und führt zur Zerstörung der lebensnotwendigen Strukturen von Mikroorganismen.

Das Frisch- oder Brauchwasser bzw. die Spülflüssigkeit wird dem hier gestrichelt angedeuteten Spülautomaten (1) über eine Frischwasserzuleitung (4) zugeführt, welche in der Regel über elektrische Magnet-und/oder Sicherheitsventile mit der örtlichen Hauswasserleitung installiert ist. Zum besseren Verständnis zeigt Fig. 2 hierfür vereinfacht den Wasserlauf des Frischwassers zum nicht näher dargestellten programmgesteuerten Spülautomaten (1). Über die Frischwasserzuleitung (4) wird die in den wasserführenden Spülprogrammabschnitten (Fig. 1) jeweils benötigte Spülflüssigkeit durch den Wasserenthärter (2) geführt und gelangt anschließend in den mit dem Spülgut geladenen Spülbehälter (5), des Spülautomaten (1).

Zur Vermeidung einer Rekontamination beim Nachspülen der Instrumente im chemothermischen Spülprozess durch das zugeführte Brauch- oder Frischwasser wird erfindungsgemäß die Spülflüssigkeit vor dem Einlauf des frischwassers in den Enthärter (2) vor der UV-Licht Bestrahlung mit einem Desinfektionsmittel auf der Basis von Peroxid zur Verstärkung der Fotooxidation angereichert bzw. beimpft. Vor ihrem Eintritt in den Spülbehälter (5), sh. Fig. 2, wird die enthärtete Spülflüssigkeit zur Keimreduzierung dann noch von der UV-Lampe bestrahlt, deren UV-Licht einen desinfizierenden Einfluss auf das Wasser durch eine Keimabtötung ausübt und das Peroxid aktiviert. Durch diese Maßnahme wird vorteilhaft die Desinfektionswirkung der Spülflüssigkeit am Spülgut optimiert und auch eine Beei n-trächtigung der Transmission des UV-Lichtes durch gelöste organische Substanzen wirksam. verhindert bzw. kompensiert.

Die erfindungsgemäß zur weiteren Desinfektion (Entkeimung) der Spülflüssigkeit vorgesehene Dosiereinrichtung (6) ist dem Wasserweg vor dem Enthärter (2) wirksam zugeordnet und ist ebenso wie die UV-Lampe von der Programmsteuerung (P) des Spülautomaten (1) ansteuerbar. Eine Desinfektion des Wassereinlaufs mit dem Desinfektionsmittel Peressigsäure (POM) bzw. Wasserstoff-Peroxid ist favorisiert, wobei bei jedem Wassereinlauf etwa 10-50ppm POM direkt vor dem Enthärter dosiert werden. Die dosierende Menge beträgt in etwa 0,2 - 1 ml pro Liter Spülflüssigkeit, d. h. bei einem Wassereinlauf von ca. 1,6 bis 8 Liter Frischwasser pro Minute. Das Desinfektionsmittel wird dabei während des gesamten Wassereinlaufs zudosiert, damit sich im Anschluss an jeder Stelle Desinfektionsmittel in der vorgegebenen Konzentration befindet.

Die Beimpfung der Spülflüssigkeit mit Desinfektionsmittel kann dabei auch impulsweise erfolgen. Hierdurch ist u. U. eine bessere Anpassung der Konzentration an unterschiedliche Wasserqualitäten und Spülprogrammschritte möglich. Die Konzentration des Desinfektionsmittels ist von unterschiedlichen Wasserqualitäten und/oder den verschiedenen Spülprogrammabschnitten abhängig gemacht, wobei das Impfen des Frischwassers mit dem Desinfektionsmittel in allen wasserführenden beheizten und/oder unbeheizten Spülprogrammabschnitten erfolgt. Die aus UV-Lampe (3) und Dosiereinrichtung (6) bestehende Desinfektionseinrichtung sichert im Zusammenwirken mit dem programmgesteuert ablaufenden chemothermischen Spülprogramm die optimale Desinfektion durch Ausschluss einer Rekontamination durch Wasserkeime. Dabei kann die Einrichtung (E), bestehend aus UV-Lampe (3), Dosiereinrichtung (6) und Enthärter (2) als eine dem Spülautomaten (1) separat vorschaltbare Wasser-Desinfektionseinheit ausgebildet sein, oder diese Einheit ist fester Bestandteil des Spülautomaten.

Durch den Einsatz des erfindungsgemäßen Verfahrens wird einerseits die Chargenzeit verkürzt, wobei u. U. sogar auf das Erwärmen der Spülflüssigkeit im Nachspülgang verzichtet werden kann. Zumindest genügt aber eine geringe Wassertemperatur von ca. 35° C, so dass die Verweildauer der Spülflüssigkeit im Spülbehälter (5) gegenüber den Nachspülzeiten bekannter Spülautomaten mit einer Spülwassertemperatur von 60° C und darüber erheblich verkürzt werden kann. Dies bedeutet schon eine beträchtliche Ersparnis an Energie und Programmlaufzeit. Die Beimpfung der Spülflüssigkeit vor dem Enthärter mit Peroxid erhöht daneben die Desinfektionssicherheit des Spülautomaten (Sicherstellung der Instrumentendesinfektion). Durch das zugeführte Wasserstoff-Peroxid oder die Peressigsäure (im ppm-Bereich) fördert das UV-Licht dann zusätzlich die Bildung von hochreaktiven Hydroxylradikalen, die chemisch für eine Desinfektion durch Oxidation sorgen. Selbstverständlich ist auch eine andere Peroxid-Rezeptur möglich.

Ein derartiges Beimpfen ist darüber hinaus auch toxikologisch unproblematisch, da z. B. bei der Hämodialyse die Desinfektion auch mit Peroxid durchgeführt wird und geringfügigste Reste verbleiben können. Der Vorteil weiterhin ist, dass das in der UV-Lichteinheit nicht abreagierte Peroxid im Wasser verbleibt und zumindest noch einen gewissen desinfizierenden Einfluss, zumindest aber einen bakteriostatischen Einfluss auf die Oberflächen des wasserführenden Bereiches der UV-Lampe hat. Untersuchungen mit Pseudomonas aeruginosa, einem Wasserbakterium einerseits und mit den Sporen B. subtilis andererseits haben ergeben, dass bei Pseudomonas aeruginosa die UV-Behandlung allein zwar schon desinfizierend wirkt, dass jedoch bei B. subtilis allein durch UV-Licht kaum eine Reduktion bewirkt wird. Die Kombination von UV- Behandlung und Peroxid-Beimpfung führt jedoch zu einer starken Reduktion dieser resistenten Sporen.

## Patentansprüche

1. Verfahren zur Entkeimung von Spülflüssigkeit, die als Frisch- oder Brauchwasser in einem programmgesteuerten Spülautomaten (1) zur chemothermischen Desinfektion von medizinischen Instrumenten, wie Endoskope oder dergl. verwendet wird,
wobei die Spülflüssigkeit jeweils in den wasserführenden Programmabschnitten Vorspülen (V), Reinigen (R), Zwischenspülen (Z) und Instrumentendesinfektion oder Nachspülen (N) dem Spülbehälter (5) des Automaten (1) über einen Enthärter (2) zugeführt und mit UV-Licht bestrahlt wird,
**dadurch gekennzeichnet,**
**dass** die Spülflüssigkeit bevor sie dem Spülbehälter des Automaten für die wasserführenden Programmschritte zugeführt wird,
- zunächst in einem ersten Verfahrensschritt mit einem Desinfektionsmittel auf der Basis von Peroxid zur Verstärkung der Fotooxidation angereichert bzw. beimpft wird, und
- nach Durchfluss durch den Enthärter, zweiter Verfahrensschritt,
- in einem dritten und letztem Verfahrensschritt die angereicherte Spülflüssigkeit mit UV-Licht bestrahlt wird,
- und wobei mittels der Programmsteuerung (P) eine Dosiereinrichtung (6) für das Desinfektionsmittel sowie eine UV-Lampe für das UV-Licht angesteuert wird.

2. Verfahren nach Ansprüch 1,
**dadurch gekennzeichnet,**
**dass** die zu dosierende Desinfektionsmittelmenge über die Zeit des gesamten Frischwassereinlaufs verteilt der Spülflüssigkeit zugegeben wird.

3. Verfahren nach mindestens einem der Ansprüche 1 bis 2,
**dadurch gekennzeichnet,**
**dass** die Desinfektionsmittelzugabe zur Spülflüssigkeit impulsweise erfolgt.

4. Verfahren nach mindestens einem der Ansprüche 1 bis 3,
**dadurch gekennzeichnet,**
**dass** die Konzentration des Desinfektionsmittels in einem toxikologisch unbedenklichen Konzentrationsbereich von unterschiedlichen Wasserqualitäten und/oder den verschiedenen Spülprogrammabschnitten abhängig gemacht ist.

5. Verfahren nach mindestens einem der Ansprüche 1 bis 4,
**dadurch gekennzeichnet,**
**dass** der Spülprogrammabschnitt Nachspülen (N) als Kalt- oder Warmspülgang durchgeführt wird.

6. Verfahren nach mindestens einem der Ansprüche 1 bis 5,
**dadurch gekennzeichnet,**
**dass** als Desinfektionsmittel Peressigsäure oder Wasserstoffperoxid in einer Konzentration von vorzugsweise 10 bis 50 ppm pro Liter Spülflüssigkeit bei jedem Frischwassereinlauf einstellbar ist.

7. Spülautomat zur Durchführung des Verfahrens zur Entkeimung einer Spülflüssigkeit, die als Frisch-oder Brauchwasser in einem programmgesteuerten Ablauf zur chemothermischen Desinfektion von medizinischen Instrumenten, wie Endoskope oder dergl. Verwendung findet, wobei die Spülflüssigkeit jeweils in den wasserführenden Programmabschnitten Vorspülen (V), Reinigen (R), Zwischenspülen (Z) und Instrumentendesinfektion oder Nachspülen (N), dem Spülbehälter (5) des Automaten (1) über einen Enthärter (2) strömt und mit UV-Licht bestrahlt wird,
**dadurch gekennzeichnet,**
**dass** im Spülautomaten (1) im Bereich der Frischwasserzuleitung (4), bevor das Frischwasser in den Spülbehälter des Automaten für die wasserführenden Programmschritte einströmt, zunächst eine Dosiereinrichtung (6) angeordnet ist, die ein Desinfektionsmittel auf der Basis von Peroxid zur Verstärkung der Fotooxidation der Spülflüssigkeit zudosiert, und der Dosiereinrichtung (6) der Enthärter (2) nachgeschaltet ist, wobei anschließend UV-Ucht die angereicherte Spülflüssigkeit bestrahlt, und wobei sowohl die Dosiereinrichtung (6) als auch die UV-Lampe (3) von der Programmsteuerung (P) des Spülautomaten (1) ansteuerbar ist.

## Claims

1. Method for sterilising rinsing fluid, which is used as fresh or industrial water in a program-controlled automatic washer (1) for the chemothermal disinfection of medical instruments, such as endoscopes or the like, in which the rinsing fluid is supplied to the wash container (5) of the automatic washer (1) via a softener (2) in each of the water-carrying program sections pre-rinse (V), cleaning (R), intermediate rinse (Z) and instrument disinfection or final rinse (N), and is irradiated with UV light,
**characterised in that**
before the rinsing fluid is supplied to the wash container of the automatic washer for the water-carrying program steps, it is
- initially enriched or inoculated with a peroxide-based disinfectant to increase photo-oxidation, in a first method step, and
- after flowing through the softener, the second method step,
- the enriched rinsing fluid is irradiated with UV light in a third and last method step,
- and a metering device (6) for the disinfectant and a UV lamp for the UV light being controlled by means of the programmed control (P) system.

2. Method according to claim 1, **characterised in that** the amount of disinfectant to be metered is added to the rinsing fluid distributed over the time of the total inflow of fresh water.

3. Method according to at least one of claims 1 and 2, **characterised in that** the disinfectant is added to the rinsing fluid in a pulsed manner.

4. Method according to at least one of claims 1 to 3, **characterised in that** the concentration of the disinfectant in a toxicologically safe concentration range is made dependent on different water qualities and/or the various rinsing program sections.

5. Method according to at least one of claims 1 to 4, **characterised in that** the rinsing program section final rinse (N) is carried out as a cold or hot rinse cycle.

6. Method according to at least one of claims 1 to 5, **characterised in that**, as the disinfectant, peracetic acid or hydrogen peroxide may be used in a concentration of preferably 10 to 50 ppm per litre rinsing fluid during each inflow of fresh water.

7. Automatic washer for carrying out the method of sterilising a rinsing fluid which is used as fresh or industrial water in a program-controlled sequence for the chemothermal disinfection of medical instruments, such as endoscopes or the like, in which the rinsing fluid is supplied to the wash container (5) of the automatic washer (1) via a softener (2) in each of the water-carrying program sections pre-rinse (V), cleaning (R), intermediate rinse (Z) and instrument disinfection or final rinse (N), and is irradiated with UV light,
**characterised in that**
in the automatic washer (1) in the region of the fresh water supply line (4) before the fresh water flows into the wash container of the automatic washer for the water-carrying program steps, there is first arranged a metering device (6) which meters a peroxide-based disinfectant into the rinsing fluid to increase photo-oxidation, and the softener (2) is downstream of the metering device (6), UV light then irradiating the enriched rinsing fluid, and both the metering device (6) and the UV lamp (3) being controllable by the programmed control (P) system of the automatic washer (1).

## Revendications

1. Procédé de stérilisation d'un liquide de rinçage ou de nettoyage que l'on utilise sous forme d'eau potable ou d'eau industrielle dans une machine à laver automatique programmable (1) pour la désinfection chimiothermique d'instruments médicaux, tels que des endoscopes ou équivalents, dans lequel le liquide de rinçage ou de nettoyage est chaque fois amené au compartiment de rinçage (5) de la machine à laver automatique (1), en passant par un dispositif adoucisseur (2), au cours des séquences du programme amenant de l'eau, pré-lavage (V), lavage (R), rinçage intermédiaire (Z) et désinfection des instruments ou rinçage ultérieur (N), et irradié à l'aide d'un rayonnement ultraviolet, **caractérisé en ce que**
le liquide de rinçage ou de nettoyage, avant d'être amené au compartiment de rinçage de la machine à laver automatique pour les séquences de programme amenant de l'eau,
- se voit, au cours d'une première étape du procédé, d'abord inoculé ou enrichi d'un produit désinfectant à base de peroxyde pour renforcer la photo-oxydation, puis
- après avoir traversé le dispositif adoucisseur en deuxième étape du procédé,
- le liquide de rinçage ou de nettoyage enrichi est, au cours d'une troisième et dernière étape du procédé, irradié par un rayonnement ultraviolet,
et dans lequel l'unité de commande programmable (P) permet de commander un dispositif de dosage (6) du produit désinfectant, ainsi qu'une lampe à U.V. pour la lumière ultraviolette.

2. Procédé selon la revendication 1, **caractérisé en ce que** la quantité de produit désinfectant à doser est délivrée dans le liquide de rinçage ou de nettoyage de façon à être répartie sur toute la durée d'alimentation en eau potable.

3. Procédé selon la revendication 1 ou 2, **caractérisé en ce que** l'adjonction du produit désinfectant au liquide de rinçage ou de nettoyage se fait par impulsions.

4. Procédé selon au moins l'une des revendications 1 à 3, **caractérisé en ce que** la concentration du produit désinfectant, dans une gamme de concentrations sans risques sur le plan toxicologique, est rendue tributaire des différentes qualités d'eau et/ou des différentes séquences du programme de lavage.

5. Procédé selon au moins l'une des revendications 1 à 4, **caractérisé en ce que** la séquence du programme de lavage de rinçage ultérieur (N) prend la forme d'une opération de rinçage à froid ou à chaud.

6. Procédé selon au moins l'une des revendications 1 à 5, **caractérisé en ce que** la quantité de produit désinfectant sous forme d'acide peracétique ou de peroxyde d'hydrogène, en concentration de préférence comprise entre 10 et 50 ppm par litre de liquide de rinçage, peut être ajustée à chaque apport d'eau potable.

7. Machine à laver automatique pour la mise en oeuvre du procédé de stérilisation d'un liquide de rinçage ou de nettoyage utilisé sous forme d'eau potable ou d'eau industrielle dans un processus automatisé de désinfection chimiothermique d'instruments médicaux, tels que des endoscopes ou équivalents, dans lequel le liquide de rinçage ou de nettoyage est chaque fois introduit dans le compartiment de rinçage (5) de la machine à laver automatique (1), en passant par un dispositif adoucisseur (2), au cours des séquences du programme amenant de l'eau, pré-lavage (V), lavage (R), rinçage intermédiaire (Z) et désinfection des instruments ou rinçage ultérieur (N), et irradié à l'aide d'un rayonnement ultraviolet, **caractérisée en ce qu'**un dispositif de dosage (6) est d'abord installé dans la machine à laver automatique (1) dans la zone de la conduite d'amenée d'eau potable (4), avant que l'eau potable pénètre dans le compartiment de rinçage de la machine à laver automatique pour les séquences du programme amenant de l'eau, qui délivre dans le liquide de rinçage ou de nettoyage en le dosant, un produit désinfectant à base de peroxyde pour renforcer la photo-oxydation, **en ce que** le dispositif adoucisseur (2) est intercalé à la suite du dispositif de dosage (6), une lumière ultraviolette irradiant enfin le liquide de rinçage ou de nettoyage enrichi, le dispositif de dosage (6) ainsi que la lampe à U.V. (3) pouvant être commandés par l'unité de commande programmable (P) de la machine à laver automatique (1).
